# EUROPÄISCHE PATENTSCHRIFT

(11) **EP 1 459 061 B1**
(45) Veröffentlichungstag und Bekanntmachung des Hinweises auf die Patenterteilung: **28.01.2009**
(21) Anmeldenummer: 02792624.5
(22) Anmeldetag: 29.11.2002
(51) Int. Cl.: G01N 33/00

(54) **VERFAHREN ZUR BESTIMMUNG VON STICKOXYDEN IN EINEM ABGAS UND VORRICHTUNG ZUR ABGASNACHBEHANDLUNG**
METHOD FOR THE DETERMINATION OF NITROGEN OXIDES IN AN EXHAUST GAS AND DEVICE FOR EXHAUST GAS TREATMENT
PROCEDE PERMETTANT DE DETERMINER LA TENEUR EN AZOTE DE GAZ D'ECHAPPEMENT ET DISPOSITIF DE RETRAITEMENT DES GAZ D'ECHAPPEMENT

(30) Priorität: 21.12.2001 DE 10163233
(43) Veröffentlichungstag der Anmeldung: 22.09.2004
(73) Patentinhaber: ROBERT BOSCH GMBH, 70442 Stuttgart (DE)
(72) Erfinder: BREUER, Norbert, 71254 Ditzingen (DE)
(86) Internationale Anmeldenummer: PCT/DE2002/004389
(87) Internationale Veröffentlichungsnummer: WO 2003/056318

(56) Entgegenhaltungen:
- EP-A- 0 732 587
- US-A- 3 692 485
- US-A- 4 822 564
- US-A- 5 565 075
- US-A- 5 580 433
- US-B1- 6 312 585

## Beschreibung

### Stand der Technik

Die Erfindung geht von einem Verfahren zur Bestimmung von Stickoxiden in einem Abgas sowie von einer Vorrichtung zur Abgasnachbehandlung gemäß der in dem Oberbegriff der unabhängigen Ansprüche näher definierten Art aus.

Es bestehen in der Praxis hohe Anforderungen hinsichtlich von Emissionen von Verbrennungseinrichtungen bzw. -anlagen, insbesondere von Brennkraftmaschinen von Kraftfahrzeugen, was den Einsatz effizienter Abgasreinigungsverfahren bedingt. Um diese Abgasreinigungsverfahren hinreichend zuverlässig steuern und kontrollieren zu können, müssen Sensoren eingesetzt werden, mittels derer die Konzentrationen von beim Betrieb einer Verbrennungseinrichtung erzeugten Gasen möglichst genau, schnell und selektiv nachgewiesen werden können. Insbesondere beim Einsatz in einem Kraftfahrzeug müssen die Sensoren bzw. Methoden robust gegenüber mechanischen und thermischen Beanspruchungen sein, eine hinreichend lange Standzeit mit möglichst geringem Drift der Meßwerte aufweisen und zudem kostengünstig durchführbar sein.

Zum Nachweis von Stickoxiden in einem Abgas einer Brennkraftmaschine sind Sensoren bekannt, bei welchen ein elektrochemischer Reduktionsstrom ausgewertet wird, der bei der Reduktion von Stickoxid auftritt. Der Nachweis einer NOₓ-Reduktion ist insbesondere schwierig, wenn in dem Abgas zusätzlich Sauerstoff vorliegt, was bei einem Abgas einer mager betriebenen Brennkraftmaschine der Fall ist, denn neben geringen Mengen von NOₓ wird hier auch Sauerstoff reduziert, welcher in einer gegenüber den Stickoxiden wesentlich höheren Konzentration vorliegt. Dies führt zu einer unerwünschten Querempfindlichkeit. Bei einem Abgas einer Diesel-Brennkraftmaschine beträgt der Konzentrationsunterschied zwischen Stickoxiden und Sauerstoff etwa den Faktor 100 bis 1000.

Ein Verfahren und ein Sensor zur Detektion von Stickoxiden in einem Abgas durch Reduktion der Stickoxide sind aus der EP 0 845 670 A2 bekannt. Dieser Sensor umfaßt zwei Kammern, wobei aus einer Kammer zunächst Sauerstoff selektiv abgepumpt wird. Der Sensor hat einen komplexen Aufbau und ist aufgrund der Anzahl von benötigten Elektroden aufwendig herzustellen.

Aus der US 4 822 564 und der US 3 692 485 ist ein Verfahren bekannt, nach dem in einem Abgas enthaltene Stickoxide messbar sind. Das Abgas wird hierzu in eine Messkammer eingeleitet, der zusätzlich Ozon zugeführt wird, so dass eine Reaktion zwischen Stickstoffmonoxid und dem Ozon stattfindet, wobei als Folge der Reduktion Chemolumineszenz auftritt. Die Chemolumineszenz beruht auf der Tatsache, dass ein Teil des bei der Oxidation von Stickstoffmonoxid erzeugten Stickstoffdioxids zunächst auf einen angeregten Zustand erregt wird und dann unter Abstrahlung von Licht auf eine niedrigeres Energieniveau relaxiert. Das abgestrahlte Licht wird mittels einer Photodiode gemessen. Der gemessene Photostrom stellt ein Maß für die Konzentration an Stickstoffmonoxid in dem Abgas dar.

Weiterhin sind aus der US 6,312,585, der US 5,580,433, der EP 0732587 und der US 5,565,075 Verfahren zur Bestimmung von Stickstoffmonoxid beschrieben, wobei die Bestimmung des Stickstoffmonoxids mittels elektrochemischer Oxidation erfolgt.

### Vorteile der Erfindung

Die Erfindung hat auch ein Verfahren zur Bestimmung von Stickoxiden in einem Abgas mit den Merkmalen nach dem Oberbegriff des Patentanspruches 1 zum Gegenstand, bei welchem Verfahren eine Kenngröße der mittels des Oxidationsmittels ausgelösten Oxidationsreaktion als ein Maß für die Konzentration des Stickstoffmonoxids in dem Abgas ausgewertet wird. Da auch bei diesen Verfahren die Oxidationsreaktion von Stickstoffmonoxid ausgenutzt wird, ist es nicht erforderlich, in dem Abgas enthaltenen Sauerstoff durch vorherige Reduktion aus dem Abgas zu entfernen.

Der Begriff Kenngröße bezeichnet eine physikalische Größe, die unmittelbar mit der Oxidationsreaktion zwischen Stickstoffmonoxid und dem Oxidationsmittel verbunden ist und eine mit der oxidierten Menge an Stickstoffmonoxid korrelierte Größe darstellt.

Durch Einsatz der Verfahren nach der Erfindung ist die Oxidation des in einem Abgas enthaltenen Stickstoffmonoxids als Meßgröße verfolgbar. Bei bestimmen Anwendungen kann es sinnvoll sein, die Gesamtmenge an in dem Abgas enthaltenen Stickoxiden zu kennen. In vielen Fällen ist die prozentuale Verteilung der Einzelverbindungen der Stickoxide, wie Stickstoffmonoxid und Stickstoffdioxid, in engen Grenzen bekannt. Dann kann mit dem Nachweis der Oxidationsreaktion von Stickstoffmonoxid zu Stickstoffdioxid auf die Gesamtmenge an in dem Abgas enthaltenen Stickoxiden geschlossen werden.

Die Kenngröße, die als ein Maß für die Konzentration des Stickstoffmonoxids in dem Abgas ausgewertet wird, kann beispielsweise den Verbrauch an Oxidationsmittel darstellen. Der Verbrauch an Oxidationsmittel wird beispielsweise in Form einer Konzentrationsänderung über die Kontaktzeit zwischen Oxidationsmittel und Abgas festgestellt. Ein derartiges Verfahren kann entweder mittels zweier Sensoren durchgeführt werden, an welchen das Oxidationsmittel selektiv nachgewiesen werden kann und welche vorteilhaft genau definiert an zwei voneinander beabstandeten Positionen, beispielsweise im Abstand von 5 cm, entlang des Abgasstrangs stromabwärts einer Zudosierstelle für das Oxidationsmittel angeordnet sind. Über die Differenz der mittels der beiden Sensoren gemessenen Meßwerte kann der Abbau des Oxidationsmittels ermittelt werden, was wiederum ein Maß für die erfolgte Oxidationsreaktion und damit für die Stickstoffmonoxidkonzentration in dem Abgas ist. Wenn die Menge des zudosierten Oxidationsmittels bekannt ist, so ist gegebenenfalls ein Sensor ausreichend, der stromab der Zudosierstelle für das Oxidationsmittel, beispielsweise im Abstand von 5 cm von dieser, angeordnet ist.

Die Zudosierung des Oxidationsmittels kann zeitlich moduliert, insbesondere diskontinuierlich, d. h. in zeitlich begrenzten Intervallen erfolgen. Das Oxidationsmittel kann aber auch kontinuierlich zudosiert werden.

Alternativ kann die Menge an in den Abgasstrang zudosiertem Oxidationsmittel so geregelt werden, dass sich an dem Sensor zum Nachweis des Oxidationsmittels ein genau definierter Schwellwert einstellt. Erhöht sich die Stickstoffmonoxidkonzentration des Abgases, so wird bei dieser Ausführungsform die Menge an zudosiertem Oxidationsmittel erhöht, damit der Schwellwert eingehalten wird. Hierzu können Mittel zur Einstellung einer konstanten Oxidationsmittelkonzentration an dem Sensor vorgesehen sein.

Die Zudosierung des Oxidationsmittels kann aus einem im Bereich des Abgasstrangs angeordneten Speicher über ein Dosierventil erfolgen. Der Speicher enthält als Oxidationsmittel beispielsweise Wasserstoffperoxid oder Ozon.

Das Oxidationsmittel, z.B. Ozon, kann aber auch in dem Abgasstrang oder außerhalb des Abgasstrangs mittels eines sogenannten Oxidationsmittelgenerators erzeugt werden.

Die Erfindung hat auch eine Vorrichtung zur Abgasnachbehandlung mit den Merkmalen nach dem Patentanspruch 8 zum Gegenstand.

Diese Vorrichtung weist jeweils einen einfachen konstruktiven Aufbau auf, der hinsichtlich den beispielsweise bei einem Kraftfahrzeug auftretenden hohen thermischen und mechanischen Belastungen standhält.

Das Verfahren und die Vorrichtung nach der Erfindung können grundsätzlich bei jeglichen Verbrennungseinrichtungen bzw. -anlagen zum Einsatz kommen. So kann es sich bei der Verbrennungseinrichtung beispielsweise um eine Heizung oder dergleichen oder auch um eine Brennkraftmaschine handeln, die stationär ausgebildet ist oder Bestandteil eines Kraftfahrzeuges ist. Insbesondere ist die Anwendung der Erfindung sinnvoll, wenn ein mageres Abgas bezüglich Stickoxiden überwacht werden muss.

Weitere Vorteile und vorteilhafte Ausgestaltungen des Gegenstandes nach der Erfindung sind der Beschreibung, der Zeichnung und den Patentansprüchen entnehmbar.

### Zeichnung

Vier Ausführungsbeispiele des Gegenstandes nach der Erfindung sind in der Zeichnung schematisch vereinfacht dargestellt und werden in der nachfolgenden Beschreibung näher erläutert. Es zeigen
Figur 1 einen Abgasstrang, bei dem eine Stickstoffmonoxidkonzentration mittels zweier Gassensoren gemessen wird;
Figur 2 einen Abgasstrang, bei dem eine Stickstoffmonoxidkonzentration mittels eines Gassensors für Oxidationsmittel gemessen wird;
Figur 3 einen Baugruppenträger zum Einbau in einen Abgasstrang; und
Figur 4 ein Sensorelement mit einer elektrochemischen Zelle.

### Beschreibung der Ausführungsbeispiele

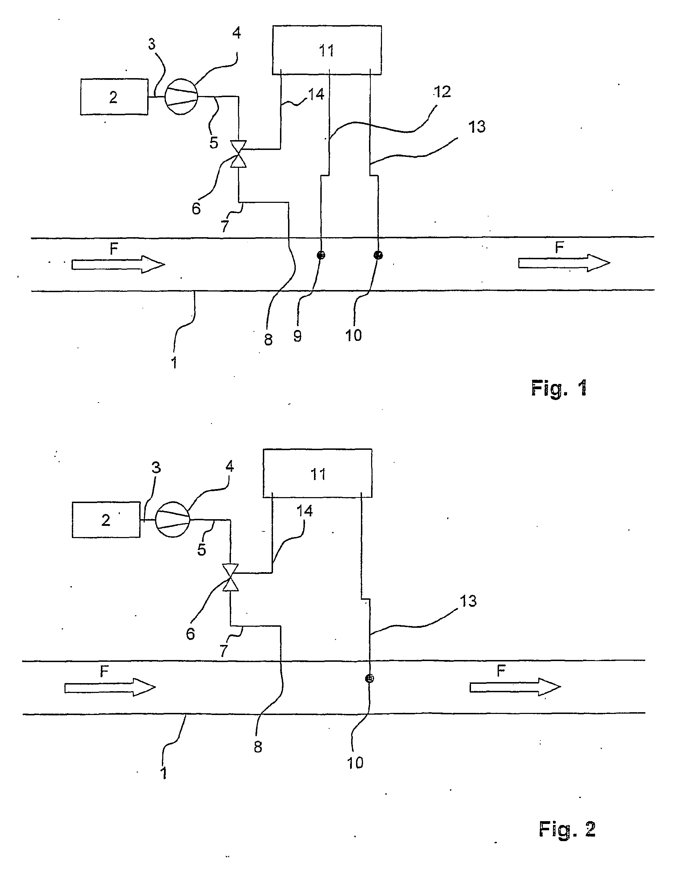

In Figur 1 ist ein Abgasstrang 1 eines hier nicht näher dargestellten Kraftfahrzeuges mit einer Diesel-Brennkraftmaschine dargestellt. In dem Abgasstrang 1 strömt ein während des Betriebs der Brennkraftmaschine entstehendes Abgas in der mit einem Pfeil F gekennzeichneten Richtung. Der Abgasstrang 1 ist mit einer Vorrichtung zur Detektion von in dem Abgas enthaltenen Stickoxiden ausgestattet.

Im Bereich des Abgasstrangs 1 ist ein Speicher 2 für ein Oxidationsmittel angeordnet, welches im vorliegenden Fall Wasserstoffperoxid oder Ozon ist. Der Oxidationsmittelspeicher 2 ist über einen Leitungsabschnitt 3 mit einer Förderpumpe 4 verbunden, welche förderseitig über einen Leitungsabschnitt 5 mit einem Dosierventil 6 verbunden ist, von dem ein Leitungsabschnitt 7 abzweigt, der in den Abgasstrang 1 mündet und dessen stromabseitiges Ende 8 eine Zudosierstelle für das Oxidationsmittel darstellt.

Das Dosierventil 6 ist des weiteren über eine Signalleitung 14 mit einem Steuergerät 11 verbunden, so daß mittels des Dosierventils 6 eine kontrollierte und gesteuerte Zudosierung von Oxidationsmittel in den Abgasstrang 1 durchgeführt werden kann.

Stromab der Zudosierstelle 8 für das Oxidationsmittel ist im Abstand von etwa 5 cm ein erster Sensor 9 zur Detektion von Oxidationsmittel und stromab von diesem, ebenfalls im Abstand von etwa 5 cm, ein zweiter Sensor 10 zur Detektion von Oxidationsmittel angeordnet. Mittels der Sensoren 9 und 10 ist die Konzentration an Oxidationsmittel an der jeweiligen Stelle in dem Abgasstrang 1 meßbar, so daß beim Vorliegen von Stickstoffmonoxid in dem Abgas der Verbrauch an über die Zudosierstelle 8 zugeführten Oxidationsmittel zwischen den Sensoren 9 und 10 ermittelbar ist. Aus dem so ermittelten Oxidationsmittelumsatz bzw. -verbrauch, der eine Kenngröße der Oxidationsreaktion darstellt, wird wiederum auf die Menge bzw. Konzentration an in dem Abgas enthaltenen Stickstoffmonoxid geschlossen. Die Auswertung der mittels der Sensoren 9 und 10 ermittelten Meßwerte erfolgt mittels der Steuereinheit 11, welche über Leitungen 12 und 13 mit dem ersten Sensor 9 bzw. mit dem zweiten Sensor 10 in Verbindung steht.

Das in Figur 2 dargestellte Ausführungsbeispiel unterscheidet sich von demjenigen nach Figur 1 dadurch, daß die Vorrichtung zur Detektion von Stickoxiden in dem in dem Abgasstrang 1 strömenden Abgas nur einen Sensor 10 zur Detektion von Oxidationsmittel aufweist. Bei dieser Ausführungsform muß die über die Zudosierstelle 8 dem Abgas zugeführte Oxidationsmittelmenge bekannt sein, so daß über die Kontaktzeit zwischen der Zudosierstelle 8 und dem Sensor 10 der Umsatz an Oxidationsmittel mit in dem Abgas enthaltenem Stickstoffmonoxid ermittelt werden kann. Der Umsatz bzw. Verbrauch an Oxidationsmittel stellt eine Kenngröße dar, die mittels der Steuereinheit 11 als ein Maß für die Konzentration des Stickstoffmonoxids in dem Abgas ausgewertet wird.

Die Zudosierung der Oxidationsmittelmenge wird bei dieser Ausführungsform mittels der Steuereinheit 11, welche mit dem Dosierventil 6 verbunden ist, gesteuert.

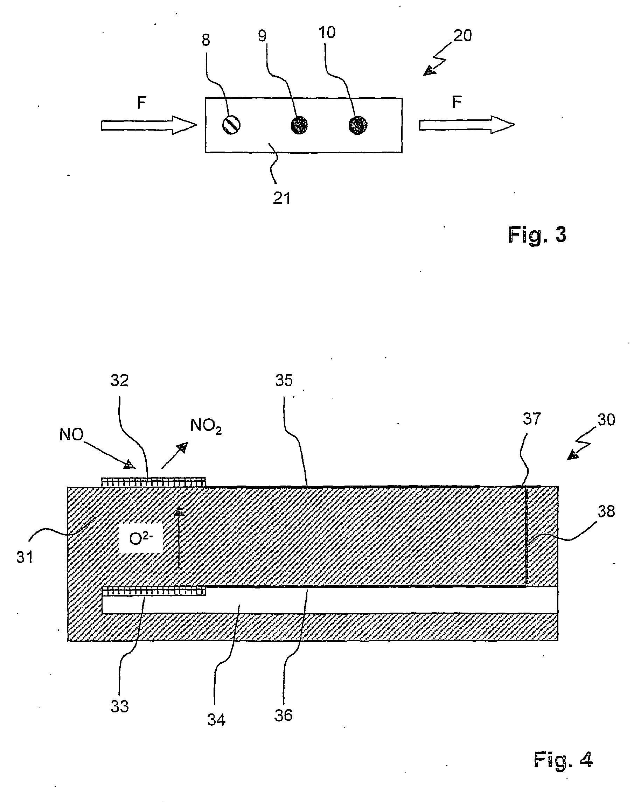

In Figur 3 ist ein Baugruppenträger 20 dargestellt, der beispielsweise zum Einsatz bei der in Figur 1 dargestellten Vorrichtung dienen kann. Der Baugruppenträger 20 umfaßt eine Grundplatte 21, die in einem Abgasstrang befestigt werden kann und an der eine mit einer entsprechenden Leitung verbundene Zudosierstelle 8, im Abstand von 5 cm stromab der Zudosierstelle 8 ein erster Oxidationsmittelsensor 9 und wiederum stromab des ersten Oxidationsmittelsensors 9 im Abstand von etwa 5 cm ein zweiter Oxidationsmittelsensor 10 angeordnet sind. Die Strömungsrichtung des Abgases ist wiederum mit einem Pfeil F gekennzeichnet.

Durch Einsatz des Baugruppenträgers 20 kann auf vorteilhafte Weise die Geometrie der auf der Grundplatte 21 angeordneten Komponenten zueinander sehr genau fixiert werden, was die Auswertung der mittels der Sensoren 9 und 10 gewonnenen Meßwerte vereinfacht.

In Figur 4 ist ein Sensor 30 zur Detektion von Stickstoffmonoxid dargestellt, der aus einer elektrochemischen Zelle gebildet ist. Der Sensor 30 umfaßt einen Keramikkörper 31, dem eine elektrochemische Zelle zugeordnet ist und der aus einem Festkörperelektrolyten 31, wie yttriumstabilisiertem Zikoniumdioxid, gebildet ist. Der Festkörperelektrolyt 31 ist O²⁻-ionenleitend.

Der Festkörperelektrolyt 31 ist an einer Seite von einer ersten Elektrode 32 begrenzt, welche einem in einem Abgasstrang einer Brennkraftmaschine strömenden Abgas ausgesetzt ist und zur Oxidation von Stickstoffmonoxid NO zu Stickstoffdioxid NO₂ dient. Zum Schutz gegen abrasive Bestandteile des Abgases ist die sogenannte Oxidationselektrode 32 gegebenenfalls mit einer porösen Schutzschicht versehen. Der Festkörperelektrolyt 31 ist an der der ersten Elektrode 32 entgegengesetzten Seite von einer zweiten Elektrode 33 zur Reduktion von Sauerstoff zu O²⁻-Ionen begrenzt. Diese sogenannte Reduktionselektrode 33 ist über einen Kanal 34 mit der Umgebungsluft verbunden.

Die Oxidationselektrode 32 und die Reduktionselektrode 33 weisen jeweils eine Zuleitung 35 bzw. 36 auf, die auf den den Festkörperelektrolyten 31, der als Träger dient, aufgedruckt sind und mit einer hier nicht dargestellten Spannungsquelle bzw. Meß- und Auswerteeinheit verbindbar sind. Die Zuleitung 36 der Reduktionselektrode 33 weist hierzu eine zu einem Kontakt 37 führende Durchkontaktierung 38 auf.

Der mittels der beiden Elektroden 32 und 33 ermittelte Oxidationsstrom bildet ein Maß für die Konzentration an Stickstoffmonoxid in dem Abgas, dem die Oxidationselektrode 32 ausgesetzt ist. Der Oxidationsstrom wird mit der Meß- und Auswerteeinheit ausgewertet.

Der in Figur 4 dargestellte Sensor 30 kann in einem geeigneten Halter angeordnet sein, so daß er sicher in einem Abgasstrang eines Kraftfahrzeuges angeordnet werden kann.

## Patentansprüche

1. Verfahren zur Bestimmung von Stickoxiden in einem Abgas, welches in einem Abgasstrang (1) einer Verbrennungsanlage, insbesondere einer Brennkraftmaschine, strömt, wobei in dem Abgas enthaltenes Stickstoffmonoxid mittels eines Oxidationsmittels oxidiert wird, wobei eine Kenngröße der Oxidationsreaktion als ein Maß für die Konzentration des Stickstoffmonoxids in dem Abgas ausgewertet wird, **dadurch gekennzeichnet, dass** die Kenngröße den Verbrauch an Oxidationsmittel darstellt.

2. Verfahren nach Anspruch 1, **dadurch gekennzeichnet, dass** der Verbrauch an Oxidationsmittel mittels mindestens eines Gassensors (9, 10) ermittelt wird.

3. Verfahren nach Anspruch 2, **dadurch gekennzeichnet, dass** die Konzentration an Oxidationsmittel an dem Gassensor (9, 10) im wesentlichen auf einem konstanten Wert gehalten wird.

4. Verfahren nach einem der Ansprüche 1 bis 3, **dadurch gekennzeichnet, dass** das Oxidationsmittel zeitlich moduliert, insbesondere diskontinuierlich, zudosiert wird.

5. Verfahren nach einem der Ansprüche 1 bis 4, **dadurch gekennzeichnet, dass** das Oxidationsmittel in dem Abgasstrang erzeugt wird.

6. Verfahren nach einem der Ansprüche 1 bis 4, **dadurch gekennzeichnet, dass** das Oxidationsmittel außerhalb des Abgasstrangs (1) erzeugt wird.

7. Verfahren nach einem der Ansprüche 1 bis 6, **dadurch gekennzeichnet, dass** das Oxidationsmittel Ozon ist.

8. Vorrichtung zur Durchführung des Verfahrens nach Anspruch 1, mit einer Einrichtung zur Oxidation von Stickstoffmonoxid mittels eines Oxidationsmittels, und mit mindestens einem Sensor (9, 10) zur Ermittlung einer Kenngröße der mittels des Oxidationsmittels ausgelösten Oxidationsreaktion von Stickstoffmonoxid zu Stickstoffdioxid, **dadurch gekennzeichnet, dass** die Kenngröße den Verbrauch an Oxidationsmittel darstellt, und dass der Sensor (9, 10) ein Gassensor zur Detektion der Konzentration des Oxidationsmittels ist.

9. Vorrichtung nach Anspruch 8, **dadurch gekennzeichnet, dass** eine Zudosierstelle (8) für das Oxidationsmittel und der mindestens eine Sensor (9, 10) auf einem Baugruppenträger (20) angeordnet sind.

10. Vorrichtung nach einem der Ansprüche 8 bis 9, **gekennzeichnet durch** Mittel zur Einstellung einer konstanten Oxidationsmittelkonzentration an dem Sensor.

## Claims

1. Method for determining nitrogen oxides in an exhaust gas which flows in an exhaust strand (1) of a combustion plant, in particular of an internal combustion engine, with nitrogen monoxide which is contained in the exhaust gas being oxidized by means of an oxidizing agent, with a characteristic variable of the oxidation reaction being evaluated as a measure for the concentration of the nitrogen monoxide in the exhaust gas, **characterized in that** the characteristic variable represents the consumption of oxidizing agent.

2. Method according to Claim 1, **characterized in that** the consumption of oxidizing agent is determined by means of at least one gas sensor (9, 10).

3. Method according to Claim 2, **characterized in that** the concentration of oxidizing agent at the gas sensor (9, 10) is held substantially at a constant value.

4. Method according to one of Claims 1 to 3, **characterized in that** the oxidizing agent is dosed in a time-modulated, in particular discontinuous fashion.

5. Method according to one of Claims 1 to 4, **characterized in that** the oxidizing agent is generated in the exhaust strand.

6. Method according to one of Claims 1 to 4, **characterized in that** the oxidizing agent is generated outside the exhaust strand (1).

7. Method according to one of Claims 1 to 6, **characterized in that** the oxidizing agent is ozone.

8. Apparatus for carrying out the method according to Claim 1, having a device for the oxidation of nitrogen monoxide by means of an oxidizing agent, and having at least one sensor (9, 10) for determining a characteristic variable of the oxidation reaction, which is triggered by means of the oxidizing agent, from nitrogen monoxide to nitrogen dioxide, **characterized in that** the characteristic variable represents the consumption of oxidizing agent, and **in that** the sensor (9, 10) is a gas sensor for detecting the concentration of the oxidizing agent.

9. Apparatus according to Claim 8, **characterized in that** a dosing point (8) for the oxidizing agent and the at least one sensor (9, 10) are arranged on a module carrier (20).

10. Apparatus according to one of Claims 8 to 9, **characterized by** means for setting a constant oxidizing agent concentration at the sensor.

## Revendications

1. Procédé pour déterminer la teneur en oxydes d'azote dans des gaz d'échappement passant dans une conduite de gaz d'échappement (1) d'une installation de combustion notamment d'un moteur à combustion interne,
selon lequel on oxyde le monoxyde d'azote contenu dans les gaz d'échappement est oxydé par un agent oxydant,
et on exploite une grandeur caractéristique de la réaction d'oxydation, comme mesure de la concentration du monoxyde d'azote dans les gaz d'échappement
**caractérisé en ce que**
la grandeur caractéristique représente la consommation en agent oxydant.

2. Procédé selon la revendication 1,
**caractérisé en ce que**
l'on détermine la consommation en agent oxydant à l'aide d'au moins un capteur de gaz (9, 10).

3. Procédé selon la revendication 2,
**caractérisé en ce qu'**
on maintient à une valeur pratiquement constante la concentration en agent oxydant dans le capteur de gaz (9, 10).

4. Procédé selon l'une des revendications 1 à 3,
**caractérisé en ce qu'**
on dose l'agent oxydant dans le temps en le modulant notamment en le dosant de façon discontinue.

5. Procédé selon l'une quelconque des revendications 1 à 4,
**caractérisé en ce qu'**
on génère l'agent oxydant dans la conduite des gaz d'échappement.

6. Procédé selon l'une quelconque des revendications 1 à 4,
**caractérisé en ce qu'**
on génère l'agent oxydant à l'extérieur de la conduite des gaz d'échappement 1.

7. Procédé selon l'une quelconque des revendications 1 à 6,
**caractérisé en ce que**
l'agent oxydant est de l'ozone.

8. Dispositif pour la mise en oeuvre du procédé selon la revendication 1, comprenant une installation d'oxydation du monoxyde d'azote à l'aide d'un agent oxydant et au moins un capteur (9, 10) pour déterminer une grandeur caractéristique de la réaction d'oxydation déclenchée par l'agent oxydant pour transformer le monoxyde d'azote en dioxyde d'azote,
**caractérisé en ce que**
la grandeur caractéristique représente la consommation en agent oxydant, et
le capteur (9, 10) est un capteur de gaz pour détecter la concentration en agent oxydant.

9. Dispositif selon la revendication 8,
**caractérisé par**
un point de dosage (8) d'agent oxydant et au moins un capteur (9, 10) installé sur un support de composants (20).

10. Dispositif selon l'une des revendications 8 et 9,
**caractérisé par**
des moyens pour régler une concentration constante d'agent oxydant dans le capteur.
